# EUROPEAN PATENT SPECIFICATION

(11) **EP 0 691 079 B1**
(45) Date of publication and mention of the grant of the patent: **14.11.2001**
(21) Application number: 95201852.1
(22) Date of filing: 06.07.1995
(51) Int. Cl.: A23L 1/00, A61K 31/715, A61K 31/22, A61K 38/00, A23L 1/30

(54) **Enteral composition for diabetic patients**
Enterales Ernährungsrezept für Diabetiker
Composition entérale pour les diabétiques

(30) Priority: 06.07.1994 US 271114
(43) Date of publication of application: 10.01.1996
(73) Proprietor: SOCIETE DES PRODUITS NESTLE S.A., 1800 Vevey (CH)
(72) Inventor: Alexander, John, Kenilworth, IL 60043 (US); Chang, Shen-Youn, Wadsworth, IL 60083 (US); Dobbie, Robert, Lincolnshire, IL 60069 (US); Grasset, Etienne, F-92100 Boulogne-Billancourt (FR); Kamarei, Ahmad Reza, Wilmette, IL 60091 (US); Laughlin, Philip, Winnetka, IL 60093 (US); Lin, Paul, Fullerton, CA 92633 (US); Melin, Christian, Winnetka, IL 60093 (US); Reddy, Sekhar, New Milford, CT 06776 (US)
(74) Representative: Vuille, Roman

(56) References cited:
- EP-A- 0 189 160
- EP-A- 0 265 772
- EP-A- 0 326 198
- EP-A- 0 484 266
- GB-A- 2 084 172
- H.-D. Belitz, W. Grosch, Lehrbuch der Lebensmittelchemie, Springer, 1992, p.146-147
- Römmp Chemie Lexikon, Editors: J. Falbe, M. Regitz, Thieme 1995, p.1343-44
- Bailey's industrial oil and fat products, Editor: D. Swern, John Wiley & Sons 1979, Volume 1, p.430-31

## Description

The present invention relates generally to compositions and methods of treating diabetes. More specifically, the present invention relates to a method for providing nutrition, or a nutritional supplement, to a patient having diabetes without substantially increasing blood glucose levels.

Diabetes Mellitus is a chronic disorder affecting carbohydrate, fat, and protein metabolism. It is characterized by hyperglycemia and glycosuria resulting from a defect or deficient insulin secretory response. The number of patients diagnosed as diabetic was estimated in 1985 to be 10 million in the United States alone, with the figure increasing at a rate of 6% per year. Two major variants of the disease exist.

One variant, seen in about 10% of all idiopathic diabetics is referred to as insulin dependent diabetes mellitus (IDDM) or juvenile onset diabetes. This variant is frequently manifested for the first time in youth and is characterized by progressive loss of insulin secretory function by beta cells of the pancreas and hence a progressive "dependency" on exogenous insulin for the maintenance of carbohydrate metabolism. (This characteristic is shared by those non-idiopathic, or "secondary" diabetics whose disorder have their origins in pancreatic disease.)

The second variant of idiopathic diabetes is referred to as non-insulin dependent diabetes mellitus (NIDDM) or adult onset diabetes mellitus. This variant accounts for the remainder of the idiopathic diabetic population.

All diabetics, regardless of their genetic and environmental backgrounds or age of onset of the disease, have in common an apparent lack of insulin or inadequate insulin function. Because transfer of glucose from the blood to the muscle and fat is insulin dependent, diabetics lack the ability to utilize glucose adequately. Further, because glycogenolysis is ordinarily inhibited by insulin, the rate of glycogenolysis is elevated in diabetics. Both of these "derangements" from normal metabolic events lead to the accumulation of glucose in the blood (hyperglycemia) to the point where renal glucose reabsorption capacity is exceeded and glycosuria occurs. A major source of energy for the diabetic becomes fatty acids derived from triglycerides stored in fatty tissue.

In the liver, fatty acids are oxidized to ketone bodies which are circulated and used as an energy source by the tissues. In the IDDM patient and sometimes the NIDDM patient, the rate of formation of the ketone bodies may exceed their rate of utilization and ketosis along with metabolic acidosis may occur.

Since tissues appear to be starving for glucose, dietary tissue sources of protein are used in glycogenesis. Anabolic processes such as synthesis of glycogen, triglycerides and proteins are "sacrificed" to catabolic activities including glycogenolysis, gluconeogenesis and mobilization of fats. Thus, the diabetic state which has its origins in a "simple" insulin defect, results in widespread metabolic disturbances having long term pathological effects on nearly all organs and tissues of the body. Indeed, the diabetic state is one of the prime contributors to deaths caused by myocardial infarction, renal failure, cerebrovascular disease, atherosclerotic heart disease, and systemic infections.

The hyperglycemic and glycosuric conditions of the diabetic disease may be treated by a manipulation of the diet, control of body weight, and regulation of physical activity. In some diabetics, particularly those suffering from NIDDM, the hyperglycemic and glycosuric conditions can be treated by oral administration of antihyperglycemic agents such as derivatives of sulfonylureas, sulfonamides, biguanides and other compounds. For diabetics suffering from IDDM and advanced NIDDM, however, therapy has focussed on administration of exogenous insulin.

Of course, the nutritional management of diabetes is of great concern. The American Diabetes Association (ADA) has published guidelines for nutritional management of diabetes mellitus. These guidelines suggest that sufficient calories be given to a patient to achieve and maintain reasonable weight.

Pursuant to the ADA (1986 Guidelines), the diet may include up to 55%-60%, based on calories, as carbohydrates. The protein content of the diet recommended for people with diabetes in approximately 12%-20% of the total calories. The fat content of the diet is recommended to be less than 30% of the total calories. Of this polyunsaturated fats are recommended up to 10%, saturated fats recommended at less than 10%, and mono-unsaturated fats will make up the remaining percentage of the fat at a range of 10%-15%.

ADA guidelines 1994 revision are more flexible regarding percentage of calories from carbohydrates, but emphasize even further the need for an adequate lipid intake.

In many situations, it may be necessary to support a diabetic patient with an enteral diet or supplement. These diets can either be administered through a nasogastric tube or other external means or provided in a liquid form that the patient drinks. Such formulations may be necessary when patients are hospitalized and/or in other situations where the patients are unable to meet necessary dietary requirements without a nutritional formulation or supplementation.

Presently, a formulation on the market for a diabetic patient is Glucerna® sold by Ross Product Division, Abbott Labs. See U.S. Patent No. 4,921,877. Glucerna® provides approximately the following macronutrient profile: protein 16.7% of the calories; carbohydrate 33.3% of the calories; fat approximately 50% of the calories. The fat in Glucerna® is provided solely through long-chain triglycerides. Specifically, the fat is provided as 85% of the calories being high oleic safflower oil and 15% of the calories being soybean oil. The caloric density of Glucerna® is approximately 1 cal/ml.

The inventors of the present invention do not believe that presently available formulations satisfactorily meet the nutritional needs of the diabetic patient.

This invention provides a composition and the use of the composition for providing nutrition, or a nutritional supplement, to a diabetic patient. Pursuant this invention, a moderate to low carbohydrate, high fat enteral formulation is provided.

Accordingly, this invention provides an enteral composition for providing nutrition to a diabetic patient without substantially increasing blood glucose levels, the composition comprising a protein source, a carbohydrate source that comprises high amylose starch, the high amylose starch comprising 25% to 75% by weight amylose and 75% to 25% of amylopectin, and a fat source that has an n-6:n-3 ratio of not more than 10 and includes medium chain triglycerides and long chain triglycerides.

In another aspect, this invention provides the use of a protein source, a carbohydrate source that comprises high amylose starch, the high amylose starch comprising 25% to 75% by weight amylose and 75% to 25% of amylopectin, and a fat source that has an n-6:n-3 ratio of not more than 10 and includes medium chain triglycerides and long chain triglycerides, in the preparation of a medicament for enterally providing nutrition to a diabetic patient without substantially increasing blood glucose levels.

The invention allows reduced insulin to metabolize the "meal" and reduces the sensitivity to dose and timing of insulin to blunt the post-prandial serum glucose excursion. Additionally, due to the content of the formulation, improvement in the patient's tolerance to the formula is achieved.

Preferably, the protein source provides about 8 to about 25% of the calories of the composition.

Preferably, the carbohydrate source provides less than 50% of the calories of the composition.

Preferably, the fat source provides about 30% to about 50% of the calories of the composition. The fat source comprises long chain triglycerides and medium chain triglycerides; the ratio of long chain triglycerides to medium chain triglycerides being preferably about 1:4.

The composition preferably includes a dietary fiber; more preferably soluble and insoluble dietary fibers.

In an embodiment, the fat source of the composition comprises approximately 40% to about 70%, by calories, mono-unsaturated fatty acids.

In an embodiment, the composition includes at least one component chosen from the group consisting of sucrose, fructose, maltose, sorbitol, or xylitol.

In an embodiment, the composition is administered to the diabetic patient through a nasogastric tube.

In an embodiment, the composition is administered as a supplementation or a sole source of nutrition to the diabetic patient.

In an embodiment, the diabetic patient is non-insulin dependent.

In an embodiment, the diabetic patient is insulin dependent.

In an embodiment, the fat source comprises, by calories, at least 4% essential fatty acids.

In an embodiment, the composition provides at least 100% of the U.S. RDA of all vitamins and minerals per 6270kJ (1500 Kcal).

An advantage of the present invention is it provides an improved composition for providing nutritional requirements and/or support to a patient having diabetes.

Furthermore, an advantage of the present invention is that it provides a method for providing nutrition to a patient having diabetes.

Additionally, an advantage of the present invention is that it provides a method and composition for providing nutrition to a diabetic patient without substantially increasing blood glucose levels.

Still further, an advantage of the present invention is that it provides a composition specifically directed to the meet the nutritional requirements and needs of the diabetic patient.

A further advantage of the present invention is to provide a composition that includes, in an embodiment, high amylose starch that is digested at a slower rate than other starches and thereby leads to a reduction in the rate at which glucose enters the blood stream.

Additionally, an advantage of the present invention is that the use of high amylose starch enhances glycemic control in the diabetic patient.

Moreover, an advantage of the present invention is to provide a fat course having an n-6:n-3 ratio that improves immune response.

Furthermore, an advantage of the present invention is to provide a formulation having dietary fiber that will tend to slow down the metabolism of carbohydrates in the formula.

Additional features and advantages of the present invention are described in, and will be apparent from, the detailed description of the presently preferred embodiments.

The present invention provides a composition and method for meeting the nutritional requirements of a diabetic patient. Pursuant to the present invention, a composition is enterally administered to the patient. The composition of the present invention is a moderate to low carbohydrate, high fat enteral formulation including a protein source, a carbohydrate source, and a fat source having a medium chain triglyceride (MCT) content. By reducing the carbohydrate intake and substituting therefor MCTs, the composition reduces the insulin necessary to metabolize the meal. Additionally, the composition reduces sensitivity to dose and timing of insulin to reduce the post-prandial serum glucose excursion. The present invention provides benefits in improved tolerance, metabolic and glucose management/insulin requirements and additional advantages.

An advantage of the present invention is provided by non-glucose, non-insulin dependent alternatives to carbohydrates in an enteral product for diabetes. The present invention avoids disadvantages of many current products and minimizes the increase in blood glucose and the associated insulin requirement which follows feeding of these patients.

In an embodiment, another advantage of the present invention is that it provides a formula including dietary fiber that will slow down the metabolism of the carbohydrates in the formula. Additionally, the use of a high amylose starch in the formula will lead to a reduction in the rate at which glucose enters the blood stream. Because it is resistant to digestion by pancreatic enzymes, the high amylose starch of the composition of the present invention enhances glycemic control in the diabetic patient. An advantage of the formula of the present invention is that it provides a composition having a low carbohydrate content and high amylose starch that is slowly digested.

The fat source preferably comprises approximately 30% to about 50% of the total calories of the composition. In a preferred embodiment, 30% to about 44% of the calories are derived from the fat source. The fat source comprises long chain triglycerides (LCTs) and medium chain triglycerides (MCTs).

MCTs are C6-C12. For example, MCTs can be a mixture of C6:0(1-2%), C8:0(65-75%), C10:0(25-30%), and C12:0(1-2%). In an embodiment, the MCTs comprise 20% of the fat source and LCTs comprise 80% of the fat source.

The use of MCTs aids in digestion. Digestion of MCTs may be easier than LCTs in that LCTs are digested by various lipases; in contrast to LCTs, pancreatic lipase is not essential to digestion of MCTs. Additionally, absorption of MCTs is faster as compared to LCTs. LCTs require incorporation into chylomicron by intestinal mucosal cells. Similarly, transport of MCTs is via portal circulation directly to the liver whereas LCTs are transported via lymphatics and systemic circulation before finally ending up in the liver. LCTs are oxidized more slowly requiring carnitine for entry into the mitochondria. The source of MCTs can comprise fractionated coconut oil.

Preferably, the LCTs are provided as canola oil, olive oil, and hi-oleic safflower oil. Although other oils can be used such as, e.g., soy oil, high-oleic sunflower oil, or any oil rich in mono-unsaturated fatty acid (MUFA). These oils not only provide linoleic acid, an essential fatty acid, but also provide n-3 fatty acids. Linolenic acid, the predominate n-3 fatty acids supplied by these oils, may serve as a precursor to other n-3 fatty acids which have anti-inflammatory activity. Preferably, at least 4%-10%, by calories, essential fatty acids are provided by the composition of the present invention.

Preferably, in an embodiment, the ratio of n-6:n-3 fatty acids is approximately 4. However, other ratios can be used with preferably the ratio of n-6:n-3 being 2 to 10. This lower ratio improves the immune response.

Additionally, the fat source comprises approximately 40% to about 70% of the total calories as mono-unsaturated fatty acids (MUFA). In a preferred embodiment, the MUFA content of the fat is approximately 58% by caloric content. This higher level of MUFA as part of a high fat/moderate carbohydrate diet provides lower serum lipids than a lower fat diet that does not contain a significant amount of MUFA. An advantage of the composition of the present invention is that it provides a high fat diet with MCTs and a high content of MUFAs.

Pursuant to the present invention, preferably the composition provides 8 to about 25% of the total calories as a protein. Preferably, the formulation contains caseinate, whey protein, or non-fat milk as the protein source.

Preferably the composition comprises approximately 30 to less than 50% in total calories as a carbohydrate and most preferably approximately 40% as a carbohydrate. A variety of carbohydrates can be utilized such as maltodextrins or other complex polysaccharides. If desired, the carbohydrates can be substituted for, in part, by fructose, sucrose, maltose, sorbitol or xylitol. This will provide non-glucose, non-insulin dependent alternatives which provide carbon skeletons to be used as an energy source.

Preferably, the carbohydrate component will comprise in part starch. Preferably, the composition will include a high amylose starch. A high amylose starch provides advantages over, for example, an acid modified starch. A high amylose starch will break down much more slowly than an acid modified starch and is digested more slowly as well. This leads to a reduction in the rate at which glucose enters the blood stream.

In a preferred embodiment, the amylose starch will consist of approximately 70% amylose and approximately 30% amylopectin.

In a preferred embodiment, the high amylose starch will comprise approximately 1% to about 5% of the composition; approximately 10 to 50 g/L of the composition. In a most preferred embodiment, the high amylose starch will comprise approximately 2.1% (23 g/L) of the composition.

Additionally, the composition, in a preferred embodiment, will include dietary fiber. The addition of dietary fiber to the formula tends to slow down the metabolism of the carbohydrates in the formula. Preferably, the composition includes approximately 8 grams to about 25 grams per liter of the composition as dietary fiber.

The dietary fiber will preferably include both soluble and insoluble fiber. Preferably, the soluble fiber will comprise approximately 50 to about 75% of the fiber present. In a most preferred embodiment, the soluble fiber will comprise approximately 70% and the insoluble fiber will comprise 30% of the total fiber content.

Preferably, the formulation will provide 100% of the U.S. RDA in 6270kJ (1500kcal) of all vitamins and minerals. Additionally, preferably, the present invention provides 4.18-6.27kJ/ml (1.0-1.5 kcal/ml).

The composition can include a surfactant. A variety of surfactants can be used such as egg yolk phospholipids, soy phospholipids, and milk phospholipids.

By way of example and not limitation, an example of the present invention will now be given. The formulation is intended for a diabetic patient. The formulation can be fed by mouth or by tube or can be used as a supplement or as a complete diet.

| EXAMPLE | | | |
|---|---|---|---|
| Nutrient | Source | Unit | **US Diabetic (Per Liter)** |
| Caloric Density | | kJ/ml (Kcal/ml) | **4.18 (1.0)** |
| **Protein** | **(18%kJ (Cal))** | **g** | **45** |
| | Caseinates | g | 45 |
| **Fat** | **(42%kJ (Cal))** | **g** | **50** |
| | MCT | g | 10 |
| | Canola oil | g | 18.5 |
| | Hi-Oleic Safflower | g | 18.0 |
| | Soy Lecithin | g | 3.5 |
| | n-6:n-3 ratio | | 3.5 |
| | MCT/LCT = 20/80 | | |
| **CHO** | **(40%kJ (Cal))** | **g** | **100** |
| | Maltodextrin | g | 45 |
| | High amylose starch | g | 23 |
| | Fructose | g | 22 |
| | Pectin | g | 3 |
| | Soy fiber | g | 5 |
| | Gum Arabic or guar | g | 7 |
| **Dietary Fiber** | | **g** | **15** |
| Flavoring | | | Vanilla |
| Lactose | | | **Lactose free** |
| Vitamin A | | IU | 4000 |
| Beta-carotene | | mg | 2 |
| Vitamin D | | IU | 320 |
| Vitamin E | | IU | 30 |
| Vitamin K | | µg | 50 |
| Vitamin C | | mg | 140 |
| Folic Acid | | µg | 400 |
| Thiamine | | mg | 2 |
| Riboflavin | | mg | 2.4 |
| Vitamin B6 | | mg | 4 |
| Vitamin B12 | | µg | 8 |
| Niacin | | mg | 28 |
| Biotin | | µg | 300 |
| Pantothenate | | mg | 14 |
| Choline | | mg | 400 |
| Calcium | | mg | 720 |
| Phosphorus | | mg | 720 |
| Magnesium | | mg | 286 |
| Iodine | | µg | 120 |
| Manganese | | mg | 3 |
| Copper | | mg | 1.5 |
| Zinc | | mg | 15 |
| Iron | | mg | 12.8 |
| Sodium | | mg | 740 |
| Potassium | | mg | 1400 |
| Chloride | | mg | 1200 |
| Selenium | | µg | 75 |
| Chromium | | µg | 125 |
| Molybdenum | | µg | 200 |
| Carnitine | | mg | 100 |
| Taurine | | mg | 100 |
| M-Inositol | | mg | 800 |

| | **Comments** | | |
|---|---|---|---|
| **Osmolality:** | 425 mOsm/kg water max. | | |
| **Viscosity:** | 0.09 kg/m sec (90 CPS) max. | | |
| **Packaging:** | cans or bags | | |
| **Processing:** | UHT aseptic or Retorting | | |

By way of comparison, a comparison of the diabetic formula of the present invention vis-a-vis Glucerna® from Ross is set forth below.

| | | **Present Invention** | **Ross Glucerna** ® | **Comments** | |
|---|---|---|---|---|---|
| **Caloric** | KJ/ml | 4.18 | 4.43 | | |
| **Density:** | (Kcal/mL) | (1) | (1.06) | | |
| **Caloric** | | | | **ADA Guideline** | **ADA Guideline** |
| **Distribution :** | | | | **(1986)** | **(1994)** |
| Protein | % | 18 | 16.7 | 12-20 | 10-20 |
| Fat | % | 42 | 50 | <30 | <10% saturated |
| | | | | (<10% saturated) | |
| CHO | % | 40 | 33.3 | 55-65 | |

| | | | | **Present Invention** | **Ross Glucerna** |
|---|---|---|---|---|---|
| **Protein Composition :** | | | | | |
| Total Protein | g/L | 45 | 41.8 | 100% Casein | 100% Casein |

| **Fat Composition :** | | | | | |
|---|---|---|---|---|---|
| Total Fat | g/L | 50 | 55.7 | | |
| MCT | g/L | 10 | 0 | MCT/LCT=20/80 Canola | No MCT Soy |
| LCT | g/L | 40 | 55.7 | hi-oleic safflower | hi-oleic safflower |

| **Fatty Acids Profile:** | | | | | |
|---|---|---|---|---|---|
| MCT | % | 18 | 0 | 7.6% of Total Energy Intake (TEI) | 0% of TEI |
| LCT | | | | | |
| Saturated | % | 7.6 | 11.2 | 3.3% of TEI | 5.6% of TEI |
| Mono- | | | | 58% | 76% |
| Unsaturated | % | 57.8 | 76.1 | of fat calories | of fat calories |
| Poly- | | | | | |
| Unsaturated | % | 17.1 | 12.7 | | |
| n6:n3 Ratio | | 3.5 | 14 | | |
| Energy from Fatty Acids (EFA) | % | 7.2 | 7.1 | EFA:7.2% of TEI | EFA:7.1% of TEI |

| **Carbohydrate Composition:** | | | | | |
|---|---|---|---|---|---|
| | | | | | Total includes |
| Total CHO's | g/L | 105 | 93.7 | | all fibers |
| Total avail-Able CHO's | g/L | 100 | 83.3 | | |
| | | | | | |
| High amylose | | | | Resistant | Contains |
| Starch | g/L | 23 | 0 | to digestion | no starch |
| Maltodextrin | g/L | 45 | 44.4 | | |
| Fructose | g/L | 22 | 17.7 | | |
| Soluble fibers Insoluble | g/L | 10 | 0.75 | | |
| Fibers | g/L | 5 | 13.65 | | |
| Lactose | | | | Lactose-free | Lactose-free |

| **Dietary Fiber Composition:** | | | | | |
|---|---|---|---|---|---|
| Total D. | | | | Soluble: Insoluble=2:1 Note:soluble fiber slows ↓ digestion | 95% insoluble |
| Fibers | g/L | 15 | 14.4 | | |
| Pectin | g/L | 3 | - | | |
| Gurn Arabic | g/L | 7 | - | | |
| Soy fiber | g/L | 5 | 14.4 | | |
| Vitamins: Meet 100% | | 5852 | 5944 | | |
| USRDA's in kJ (Kcals): | | (1400) | (1422) | | |
| Minerals: Meet 100% | | 5852 | 5944 | | |
| USRDA's in kJ (Kcals): | | (1400) | (1422) | | |
| Trace Nutrients include Cr, Mo, Se, Taurine, Carnitine, m-Inositol | | | | | |
| | | | | | |

| Chromium: | µg/L | 125 | 124 | "Cr" improves glucose tolerance | |
|---|---|---|---|---|---|
| Flavoring | | Vanilla | Vanilla | | |
| Taste | | Sl. sweet | Sl. Sweet | | |
| Osmolality | | 400 | 375 | | |

By way of example a contemplative example of the use of the formulation will now be given.

### CONTEMPLATIVE EXAMPLE

A 45 year old insulin dependent diabetic female patient is admitted to the Intensive Care Unit with severe head trauma and multiple other injuries following an auto accident. She is unconscious and will require tube feeding of an enteral formula to meet her basic and stress nutritional needs. Standard enteral tube feeding formulas with only maltodextrin will produce high levels of blood glucose, increase insulin requirements, and make her diabetes hard to control during her recovery from the accident. Standard formulas with only long chain triglycerides are less well tolerated, absorbed less well, and metabolized more slowly.

Instead of a standard enteral tube feeding formula, she is administered by tube the formula of the present invention. The formula is administered until the patient can satisfy her caloric requirements through a normal diet.

The high amylose starch and medium chain triglycerides of this invention serve to blunt the increase of blood sugar and make her diabetes easier to control. In addition, the MCTs are more easily and rapidly absorbed, and are better transported, metabolized, and utilized as an energy source with better gastrointestinal tolerance, all of which are of a significant benefit to this type of patient.

## Claims

1. An enteral composition for providing nutrition to a diabetic patient without substantially increasing blood glucose levels, the composition comprising
- a protein source,
- a carbohydrate source that comprises high amylose starch, the high amylose starch comprising 25 to 75% by weight amylose and 75% to 25% by weight of amylopectin, and
- a fat source that has an n-6:n-3 ratio of not more than 10 and includes long chain triglycerides and medium chain triglycerides.

2. An enteral composition according to claim 1 in which the protein source provides 8% to 25% of the calories of the composition.

3. An enteral composition according to claim 1 or 2 in which the carbohydrate source provides less than 50% of the calories of the composition.

4. An enteral composition according to any one of claims 1 to 3 in which the fat source provides 30% to 44% of the calories of the composition.

5. An enteral composition according to any one of claims 1 to 4 in which the fat source comprises 40% to 70% mono-unsaturated fats.

6. An enteral composition according to any one of claims 1 to 5 in which the fat source comprises a ratio of long chain triglycerides to medium chain triglycerides being 4:1.

7. An enteral composition according to any one of claims 1 to 6 in which the fat source includes an oil selected from olive oil, canola oil, high-oleic safflower oil, high-oleic sunflower oil, and mixtures thereof.

8. An enteral composition according to any one of claims 1 to 8 in which the composition further comprises soluble and insoluble dietary fiber.

9. The use of a composition according to any one of claims 1 to 8 in the preparation of a medicament for enterally providing nutrition to a diabetic patient without substantially increasing blood glucose levels.

## Patentansprüche

1. Enterale Zusammensetzung zur Versorgung eines diabetischen Patienten mit Nahrung, ohne die Blutglukosespiegel wesentlich zu erhöhen, wobei die Zusammensetzung umfaßt
- eine Proteinquelle,
- eine Kohlenhydratquelle, die eine amylosereiche Stärke umfaßt, wobei die amylosereiche Stärke 25 bis 75 Gew % Amylose und 75 bis 25 Gew % Amylopektin aufweist, und
- eine Fettquelle, die ein n-6:n-3-Verhältnis von nicht mehr als 10 aufweist und langkettige Triglyceride und mittelkettige Triglyceride einschließt.

2. Enterale Zusammensetzung nach Anspruch 1, bei der die Proteinquelle 8 bis 25 % der Kalorien der Zusammensetzung bereitstellt.

3. Enterale Zusammensetzung nach Anspruch 1 oder 2, bei der die Kohlenhydratquelle weniger als 50 % der Kalorien der Zusammensetzung bereitstellt.

4. Enterale Zusammensetzung nach irgendeinem der Ansprüche 1 bis 3, bei der die Fettquelle 30 bis 44 % der Kalorien der Zusammensetzung bereitstellt.

5. Enterale Zusammensetzung nach irgendeinem der Ansprüche 1 bis 4, bei der die Fettquelle 40 bis 70 % einfach ungesättigte Fette umfaßt.

6. Enterale Zusammensetzung nach irgendeinem der Ansprüche 1 bis 5, bei der die Fettquelle ein Verhältnis von langkettigen Triglyceriden zu mittelkettigen Triglyceriden von 4:1 aufweist.

7. Enterale Zusammensetzung nach irgendeinem der Ansprüche 1 bis 6, bei der die Fettquelle ein Öl enthält, das ausgewählt ist aus Olivenöl, Canolaöl, ölsäurereichem Safloröl, ölsäurereichem Sonnenblumenöl und Mischungen davon.

8. Enterale Zusammensetzung nach irgendeinem der Ansprüche 1 bis 8, bei der die Zusammensetzung außerdem lösliche und unlösliche diätetische Fasern aufweist.

9. Verwendung einer Zusammensetzung nach irgendeinem der Ansprüche 1 bis 8 bei der Herstellung eines Medikaments zur enteralen Versorgung eines diabetischen Patienten mit Nahrung, ohne die Blutglukosespiegel wesentlich zu erhöhen.

## Revendications

1. Composition entérale pour assurer la nutrition d'un patient diabétique, sans augmenter notablement les taux sanguins de glucose, composition qui comprend
- une source de protéines,
- une source de glucides qui comprend un amidon à haute teneur en amylose, l'amidon à haute teneur en amylose comprenant 25 à 75 % en poids d'amylose et 75 % à 25 % en poids d'amylopectine, et
- une source de matières grasses qui a un rapport n-6:n-3 non supérieur à 10 et qui comprend des triglycérides à chaîne longue et des triglycérides à chaîne moyenne.

2. Composition entérale suivant la revendication 1, dans laquelle la source de protéines fournit 8 % à 25 % des calories de la composition.

3. Composition entérale suivant la revendication 1 ou 2, dans laquelle la source de glucides fournit moins de 50 % des calories de la composition.

4. Composition entérale suivant l'une quelconque des revendications 1 à 3, dans laquelle la source de matières grasses fournit 30 % à 44 % des calories de la composition.

5. Composition entérale suivant l'une quelconque des revendications 1 à 4, dans laquelle la source de matières grasses comprend 40 % à 70 % de matières grasses mono-insaturées.

6. Composition entérale suivant l'une quelconque des revendications 1 à 5, dans laquelle la source de matières grasses comprend un rapport des triglycérides à chaîne longue aux triglycérides à chaîne moyenne égal à 4:1.

7. Composition entérale suivant l'une quelconque des revendications 1 à 6, dans laquelle la source de matières grasses comprend une huile choisie entre l'huile d'olive, l'huile de canola, l'huile de carthame à haute teneur en acide oléique, l'huile de tournesol à haute teneur en acide oléique et leurs mélanges.

8. Composition entérale suivant l'une quelconque des revendications 1 à 8, qui comprend en outre des fibres alimentaires solubles et insolubles.

9. Utilisation d'une composition suivant l'une quelconque des revendications 1 à 8 dans la préparation d'un médicament destiné à assurer par voie entérale la nutrition d'un patient diabétique, sans augmenter notablement les taux sanguins de glucose.
